# FASCICULE DE BREVET EUROPEEN

(11) **EP 2 125 442 B1**
(45) Date de publication et mention de la délivrance du brevet: **19.06.2019**
(21) Numéro de dépôt: 08761966.4
(22) Date de dépôt: 22.01.2008
(51) Int. Cl.: B60R 19/12, B60R 19/52, B62D 25/08, B60R 21/34

(54) **MODULE D'ABSORPTION DE CHOCS POUR VEHICULE AUTOMOBILE**
AUFPRALLSABSORPTIONSMODULE FÜR KRAFTFAHRZEUG
SHOCK ABSORPTION MODULE FOR AN AUTOMOBILE

(30) Priorité: 23.01.2007 FR 0752842
(43) Date de publication de la demande: 02.12.2009
(73) Titulaire: Compagnie Plastic Omnium, 69007 Lyon (FR)
(72) Inventeur: GINJA, Stéphane, F-01500 Amberieu En Bugey (FR); CHARNAUX, Sébastien, F-01250 Tossiat (FR); GILOTTE, Philippe, F-01470 Benonces (FR)
(74) Mandataire: Remy, Vincent Noel Paul
(86) Numéro de dépôt international: PCT/FR2008/050099
(87) Numéro de publication internationale: WO 2008/110709

(56) Documents cités:
- EP-A1- 1 067 039
- EP-A1- 1 698 520
- EP-A2- 1 036 715
- EP-A2- 1 352 810
- DE-C- 613 939
- FR-A- 2 821 817
- FR-A1- 2 840 573

## Description

### Domaine technique.

La présente invention concerne le domaine des absorptions de chocs dans les véhicules automobiles, et en particulier de chocs avant de type chocs assurance.

### Etat de la technique.

En matière de chocs, les constructeurs automobiles s'imposent un cahier des charges qui envisage quatre principales catégories d'impacts, à savoir :
- Les chocs de type haute vitesse, qui correspondent à un impact frontal ou arrière du véhicule contre un obstacle rigide ou déformable. Pour un choc frontal, la vitesse du véhicule est d'au moins 56 km/h, avec un objectif de protection des occupants du véhicule.
- Les chocs de type assurance, à environ 15 km/h contre un mur fixe, tel que celui connu sous le nom de Danner ou encore AZT, ou à environ 8 km/h contre un angle de mur ou contre un poteau (chocs poteau, norme IIHS), traités avec un objectif de limitation des dégâts et coûts de réparation associés.
- Les petits chocs, ou chocs de type parking, à une vitesse inférieure à 8 km/h, traités avec un objectif de non détérioration extérieure de la peau de pare-chocs.
- Les chocs piéton réglementaires (jambe, fémur et tête).

Un système d'absorption de chocs décrit dans le document US 6 467 822 permet notamment d'empêcher, lors d'un choc assurance, la déformation des longerons, afin notamment d'épargner le radiateur et les autres organes placés derrière lui. Ce système comprend un élément transversal supérieur, prenant appui sur une poutre de chocs, capable d'absorber de l'énergie par compression contre la poutre de chocs. Cette poutre de chocs prend elle-même appui sur deux longerons avant du véhicule. L'absorption d'énergie se fait par compression de l'élément transversal.

Un autre système de pare-chocs décrit dans le document FR 2 840 573 permet d'absorber l'énergie de chocs de type piéton.

### Problème posé par l'état de la technique.

Afin d'absorber une énergie suffisante en cas de chocs assurance, il est nécessaire que l'élément transversal absorbe une majorité de l'énergie du choc, et donc que sa profondeur dans la direction longitudinale du véhicule soit supérieure à une valeur minimale, ce qui impose un certain porte-à-faux du véhicule dans la direction longitudinale, c'est-à-dire une certaine longueur de la partie avant du véhicule entre les longerons et la peau de pare-chocs.

Il résulte de l'augmentation du porte-à-faux que la géométrie extérieure du véhicule est limitée.

### Solution proposée.

L'invention vise à fournir un ensemble d'un module d'absorption de chocs pour véhicule automobile et d'une pièce structurelle inférieure du véhicule automobile selon la revendication 1.

### Avantages particuliers.

Grâce au cadre absorbeur formé par les éléments supérieur et inférieur et par les jambages, l'absorption d'énergie n'est pas assurée uniquement par l'élément transversal supérieur combiné à la poutre de chocs, lesquels sont agencés à hauteur des longerons du véhicule, mais également par des éléments agencés à une hauteur inférieure à celle des longerons, notamment grâce à la compression de l'élément transversal inférieur ou des jambages contre ladite pièce structurelle inférieure du véhicule. Il en ressort que l'absorption d'énergie par la compression d'éléments est assurée sur plusieurs surfaces d'appui et que la surface totale de compression d'absorbeurs est plus grande que dans l'état de la technique. Les surfaces d'appui comprennent ainsi non seulement l'extrémité des longerons (telle qu'une platine de fixation de la poutre de chocs), mais également une surface d'appui offerte par la pièce structurelle inférieure. Comme la surface totale d'appui est plus élevée, l'épaisseur des éléments d'absorption dans la direction longitudinale peut être diminuée, tout en pouvant absorber une énergie suffisante en cas de chocs, notamment en cas de choc assurance.

On notera que la pièce structurelle inférieure est configurée pour ne pas se déformer de façon irréversible lors d'un choc de type assurance, c'est-à-dire qu'elle demeure intacte à l'issue d'un tel choc. Cette pièce se distingue donc d'une pièce d'appui qui serait destinée à résister uniquement à un choc piéton. Ainsi, la pièce structurelle inférieure reste intacte pour un effort pouvant aller jusqu'à 30 000 Newton.

Par ailleurs, grâce au cadre absorbeur, on améliore la compatibilité entre les véhicules en offrant une surface d'absorption plus importante qu'un système d'absorption classique. Les structures rigides des véhicules ne risquent ainsi pas de constituer des pièces intrusives particulièrement dangereuses et destructrices pour l'autre véhicule. Ainsi, le cadre absorbeur, du fait de son étendue dans la direction verticale, offre une surface de chocs plus grande à l'avant du véhicule, si bien qu'en cas de collision, la probabilité pour rencontrer le système d'absorption d'énergie de l'autre véhicule est particulièrement élevée.

Par ailleurs, grâce à la paroi formant un guide d'air s'étendant depuis l'élément transversal supérieur vers le radiateur, une fonction particulièrement avantageuse pouvant être apportée par le module d'absorption consiste à guider l'air entrant par des ouvertures, ménagées dans la peau de pare-chocs, vers le radiateur du véhicule. En effet, comme le module d'absorption se trouve en avant du radiateur, il est capable d'assurer une jonction entre la peau de pare-chocs et le radiateur du véhicule pour guider l'air. Par la forme de cadre du cadre absorbeur, auquel est associée la paroi formant guide d'air s'étendant depuis l'élément transversal supérieur, le module d'absorption peut définir un compartiment de guidage de l'air, particulièrement avantageux du fait qu'il n'est pas nécessaire de rapporter sur le véhicule différentes pièces spécifiques au guidage de l'air et qu'il n'est donc pas nécessaire de gérer des jeux occasionnés par ces pièces. Ainsi, les inventeurs à la base de la présente invention ont eu l'idée de réunir sur un seul module deux domaines techniques traditionnellement dissociés dans la construction automobile, à savoir le domaine de l'absorption d'énergie et celui de l'aéraulique.

Ainsi, on dispose d'un système d'absorption sous forme d'un module pré-assemblé comportant un cadre absorbeur et une paroi formant guide d'air, que l'on peut rapporter sur le véhicule, limitant le nombre de pièces rapportées au cours du montage du véhicule. Ce module peut intégrer une multiplicité de fonctions : la fonction de traitement de chocs, notamment les chocs piétons (via les éléments transversaux inférieur et supérieur), les chocs de type parking et assurance, la fonction de renforcement de différentes pièces du véhicule, ainsi que la fonction de support d'autres organes fonctionnels du véhicule, tels que des faisceaux, des capteurs, des fixations, un avertisseur sonore, etc.

### Modes de réalisation particuliers.

Le module d'absorption définit un caisson de guidage de l'air vers le radiateur, délimité par la paroi intérieure de chacun des jambages, la paroi supérieure de l'élément transversal inférieur, et ladite paroi formant guide d'air s'étendant depuis l'élément transversal supérieur, chacune de ces parois s'étendant jusqu'au radiateur.

Eventuellement, le module d'absorption comporte en outre des moyens de guidage de l'air destinés à être agencés entre la poutre de chocs et le radiateur, de préférence venus de moulage avec la paroi formant guide d'air s'étendant depuis l'élément transversal supérieur. De tels moyens de guidage peuvent être composés de languettes repliables derrière la poutre de chocs. Ils permettent ainsi de boucher l'espace entre le radiateur et la partie arrière de la poutre, espace qui est dû généralement à la présence de crash boxes sur les parties de la poutre situées au droit des longerons.

De façon encore plus avantageuse, le caisson de guidage de l'air est un caisson d'étanchéité, apte à guider l'air entrant vers le radiateur de façon étanche. En effet, comme les éléments assurant le guidage de l'air sont tous intégrés dans le module d'absorption, en étant de préférence venus de moulage, on peut obtenir un caisson agencé pour limiter au maximum les fuites d'air (ce caisson présentant moins de 5% de fuites d'air) entre la (les) entrée(s) d'air de la peau de pare-chocs et le radiateur. Ainsi, on améliore considérablement l'effet de guidage de l'air. On peut envisager en outre de minimiser les dimensions du système de refroidissement, notamment diminuer la hauteur du radiateur, de façon qu'il soit disposé à une hauteur éventuellement inférieure à celle des longerons, ce qui pourrait permettre dans les cas les plus favorables de positionner le radiateur en-dessous de la poutre de chocs, et ainsi diminuer le porte-à-faux du véhicule dans la direction longitudinale.

L'invention peut en outre comporter l'une ou plusieurs des caractéristiques suivantes.
- Les éléments transversaux inférieur et supérieur, les jambages et la paroi formant guide d'air sont en matière plastique, de préférence venus de moulage. On comprend que ce mode de réalisation est très avantageux du point de vue des coûts de fabrication, d'assemblage, pour traiter avec une seule pièce une majorité des cahiers des charges d'absorption d'énergie, notamment pour les chocs piéton et assurance, et également pour assurer l'étanchéité de l'air guidé par le module d'absorption. Fabriquer une unique pièce pour assurer toutes ces fonctions présente en particulier l'avantage d'engendrer très peu de tolérances de fabrication.
- L'élément transversal inférieur comprend un convergent inférieur, de préférence venu de moulage. Ainsi, on utilise le module d'absorption pour assurer en outre une fonction de guidage de l'air passant sous le bloc moteur pour assurer l'aérodynamique du véhicule.
- Chaque jambage est destiné à prendre appui contre une pièce structurelle comprenant une portée d'appui, s'étendant vers le bas dans le prolongement de l'extrémité de chacun des longerons. Ainsi, les jambages participent à l'absorption par compression contre cette portée d'appui. De préférence, la pièce structurelle comporte un haubanage, par exemple sous forme d'équerre, qui reporte sur une face inférieure ou latérale du longeron, ou bien sur une pièce supplémentaire liée aux longerons, les efforts subis lors de la compression de l'un des jambages. Ce haubanage assure un renfort de la pièce d'appui, permettant de mieux résister au pliage ou à une autre déformation en cas de chocs de type assurance.
- La pièce structurelle comprend des longerons inférieurs, également appelés longeronnets ou prolonges de berceau de véhicule automobile, servant d'appui pour la compression de l'élément transversal inférieur au droit des longerons inférieurs, ou encore pour la compression de la partie inférieure des jambages. Ainsi, le cadre absorbeur prend appui non seulement sur une voie haute du véhicule, composée notamment des longerons principaux, mais également sur une voie basse du véhicule.
- Chacun des éléments transversaux supérieur et inférieur et chaque jambage comprend des moyens d'absorption de chocs de type assurance. Ainsi, chacun de ces moyens est capable de se déformer à la suite d'un tel choc, afin d'empêcher la déformation plastique du châssis du véhicule (comprenant notamment les longerons supérieurs et éventuellement les longerons inférieurs, etc.), ou encore la déformation plastique du radiateur et d'autres éléments rapportés autour de ce radiateur. On entend par déformation plastique, une déformation irréversible à la suite du choc. On notera que des moyens d'absorption de chocs de type assurance se distinguent de moyens d'absorption de chocs de type parking, lesquels sont capables d'absorber une énergie relativement faible en se déformant, ou encore de moyens de protection du piéton, destinés à traiter des chocs de type "choc jambe" ou "choc hanche", ou encore "choc tête". L'homme du métier sait distinguer de tels moyens d'absorption de chocs de type assurance. En tout état de cause, on peut considérer que le module, comprenant le cadre absorbeur et le renfort central, est capable d'assurer, en se déformant, au moins 30% de l'absorption d'énergie en cas de chocs de type assurance.
- La module d'absorption comporte, au-dessus de l'élément transversal supérieur, un renfort central. Grâce à ce renfort, que l'on pourrait également appeler plastron, on peut soutenir la partie supérieure de la peau de pare-chocs dans la zone située au-dessus des longerons, tout en assurant une fonction de renforcement de cette peau, notamment une fonction d'anti-cloquage, ainsi qu'une fonction de gestion des jeux et affleurements. Ce renfort peut également assurer une fonction de traitement de choc piéton, tel que celui appelé « choc hanche ». Le renfort central est un support de la peau de pare-chocs du véhicule.
- Le renfort central comprend la paroi formant guide d'air s'étendant depuis l'élément transversal supérieur vers le radiateur. On obtient ainsi de façon simple un module assurant des fonctions d'absorption de chocs, d'aéraulique et de support de pièce de carrosserie.
- Le module comprend des moyens amovibles d'obturation d'une entrée d'air, ménagée de préférence dans la peau de pare-chocs. Selon un mode de réalisation, ces moyens amovibles sont destinés à obturer le caisson de guidage présenté plus haut, défini par le module d'absorption. De préférence, ces moyens d'obturation comportent des volets pivotants, pouvant prendre une position d'obturation totale, dans laquelle les volets sont orientés de façon à définir ensemble une surface pleine, ne laissant pas passer d'air, et une ou plusieurs positions d'ouverture, dans lesquelles les volets définissent une surface plus ou moins ajourée, laissant passer de l'air. Les volets sont généralement pilotés de façon automatique, par exemple par des moyens motorisés ou un électro-aimant, en fonction de la vitesse du véhicule. Par exemple, on peut prévoir que les moyens d'obturation prennent une position d'obturation totale lorsque le véhicule est à l'arrêt ou fonctionne à faible puissance, et une position ouverte ou semi-ouverte à partir d'une certaine puissance.

L'invention a également pour objet un ensemble d'une poutre de chocs, d'une pièce structurelle inférieure et d'un module tel que défini ci-dessus.

### Description des figures.

L'invention sera mieux comprise à la lecture de la description qui va suivre, donnée uniquement à titre d'exemple et faite en se référant aux dessins dans lesquels :
- la figure 1 est un schéma en perspective de parties avant d'un véhicule comportant un module selon un premier mode de réalisation de l'invention,
- la figure 2 est une vue arrière en perspective d'un module d'absorption similaire à celui de la figure 1, avant son montage sur le véhicule ;
- la figure 3 est une vue similaire à la figure 2, après montage du module d'absorption sur le véhicule ;
- la figure 4 est un schéma en coupe d'un module légèrement différent de celui de la figure 1, monté sur le véhicule,
- la figure 5 est une vue partielle arrière du module d'absorption de la figure 2, selon un angle de vue différent ;
- la figure 6 est une vue partielle de dessous du module d'absorption de la figure 3, selon un angle de vue différent ;
- la figure 7 est un schéma en perspective de parties avant d'un véhicule comportant un module selon un deuxième mode de réalisation de l'invention,
- la figure 8 est un schéma en coupe d'un module légèrement différent de celui de la figure 7,
- la figure 9 est une vue de face d'une partie du module de la figure 8,
- la figure 10 est une vue similaire à celle de la figure 1, le module d'absorption comprenant des moyens amovibles d'obturation d'une entrée d'air, en position d'obturation totale, et
- la figure 11 est une vue similaire à celle de la figure 10, les moyens amovibles d'obturation étant en position ouverte.

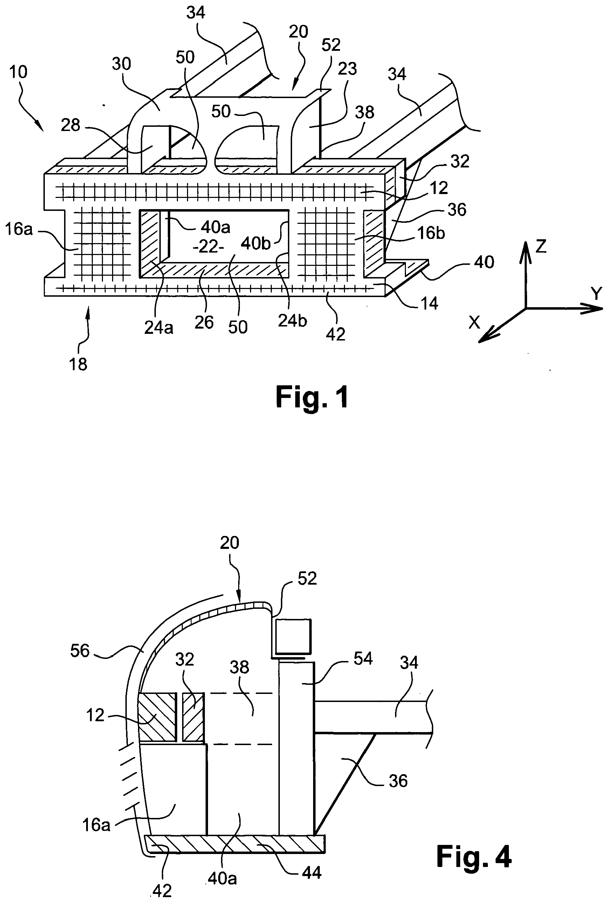

Comme on peut le voir sur le schéma de la figure 1, un module 10 d'absorption de chocs comporte un élément transversal supérieur 12, un élément transversal inférieur 14, et deux jambages 16a, 16b reliant les éléments transversaux supérieur 12 et inférieur 14 de façon à former un cadre absorbeur 18. Le module 10 est réalisé en matière plastique, de préférence en polypropylène. Chacun des éléments 12, 14, 16a et 16b du cadre absorbeur 18 comporte des moyens d'absorption de chocs de type assurance. De façon à optimiser l'absorption, ces moyens comprennent des nervures disposées en nid d'abeille.

Le module 10 comporte également un renfort central 20, également appelé "plastron", s'étendant au-dessus de l'élément supérieur 12, ayant une forme concave capable d'épouser sensiblement la forme intérieure de la peau de pare-chocs 56 du véhicule, comme on peut le voir sur la figure 4, de façon à renforcer cette dernière, notamment en la soutenant et en exerçant une fonction d'anti-cloquage de la peau. On notera que ce renfort 20 peut également servir de support pour une autre pièce de carrosserie qu'une peau de pare-chocs, et qu'il est capable de participer à la gestion des jeux et affleurement entre différentes pièces de carrosserie, par exemple le haut de la peau de pare-chocs avec le capot. Ce renfort 20 est également réalisé en matière plastique, venu de moulage avec le cadre absorbeur 18.

Le module 10 définit un caisson 22 de guidage de l'air vers le radiateur du véhicule. Ce caisson 22 est délimité notamment par la paroi intérieure 24a et 24b de chacun des jambages 16a, 16b, par la paroi supérieure 26 de l'élément transversal 14, ainsi que des parois intérieures du renfort central 20, comprenant des parois verticales 28 et une paroi sensiblement horizontale 30. La paroi 30 notamment forme un guide d'air s'étendant depuis l'élément transversal supérieur vers le radiateur du véhicule. Chacune des parois 28, 30 s'étend jusqu'au radiateur lorsque le module est monté sur le véhicule, éventuellement à l'aide d'une paroi de prolongement.

Une poutre de chocs 32 est rapportée sur le module 10, de façon à servir d'appui pour la compression de l'élément transversal supérieur 12. Cette poutre de chocs 32 comporte éventuellement, sur sa face arrière, deux absorbeurs ou crash-boxes, rapportés ou d'un seul tenant, disposés au droit des longerons 34 du véhicule.

Le module 10 et la poutre de chocs 32 sont rapportés sur le châssis avant du véhicule. Ce châssis comporte, en plus des deux longerons supérieurs 34, deux pièces structurelles inférieures 36, s'étendant chacune vers le bas dans le prolongement de l'extrémité de chacun des longerons 34, présentant une portée d'appui, de façon à servir de surface d'appui aux jambages 16a et 6b, et éventuellement aux parties de l'élément transversal inférieur 14 situées en dessous des longerons. Ces pièces structurelles inférieures 36 comportent chacune un haubanage en forme d'équerre, capable de reporter sur la face inférieure des longerons 34 les efforts subis lors de la compression des jambages 16a, 16b, ou lors de la compression de l'élément inférieur 14 contre la portée d'appui de chaque pièce 36. Ces pièces structurelles 36 sont suffisamment résistantes pour demeurer intactes sur le véhicule en cas de choc de type assurance, c'est-à-dire qu'elles ne se déforment pas de façon plastique.

Ainsi, en cas de choc assurance, les éléments 12; 14, 16a et 16b du module 10 sont capables de se déformer, par compression contre les longerons 34 et la portée d'appui des pièces structurelles 36, de façon à absorber l'énergie du choc. Cette énergie peut par ailleurs être absorbée par la compression des crash-boxes agencées sur la poutre de chocs 32. Grâce à cette absorption de chocs, le châssis du véhicule n'est pas déformé par un choc de type assurance et en conséquence, le radiateur agencé derrière le module, entre les deux longerons 34, n'est pas endommagé par le choc.

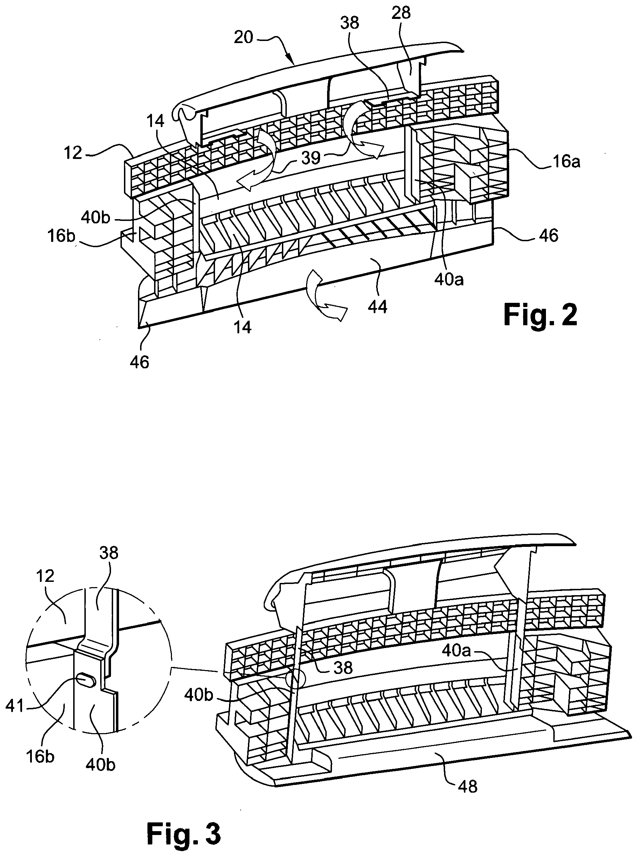

Pour le cas, représenté sur la figure 1, où la poutre de chocs 32 comporte des crash-boxes (non visibles), le caisson de guidage 22 est complété par des languettes 38, venues de moulage avec les parois latérales 28 du renfort central 20, capables de se rabattre derrière la poutre de chocs 32 lorsque cette dernière est rapportée sur le module 10, comme cela est illustré par les flèches 39 de la figure 2. De telles languettes 38 sont reliées aux parois latérales 28 au moyen d'un film charnière, de façon à pouvoir être démoulées et dépliées facilement pour former un guide d'air entre la poutre de chocs 32 et le radiateur, visible sur la figure 3. Elles comportent des moyens de fixation 41 à des guides d'air inférieurs 40a et 40b, lesquels prolongent les parois intérieures 24a et 24b, de façon à combler l'espace formé entre les jambages 16a et 16b et le radiateur, et ainsi fermer le caisson de guidage 22. Les moyens de fixation des languettes 38 sur les guides d'air 40a et 40b sont de préférence amovibles, et se présentent par exemple sous la forme de clips.

Par ailleurs, l'élément transversal inférieur 14 comprend, sur sa face tournée vers la peau de pare-chocs, un appui bas 42, destiné à traiter le choc piéton appelé choc jambe, ainsi que, venu de moulage avec cet appui bas, un convergent inférieur 44, destiné à guider l'air vers le radiateur. On notera que l'appui bas 42 constitue un appui pour la jambe d'un piéton en cas de choc, afin d'épargner son genou. Afin de rigidifier cet appui bas 42, on prévoit des nervures sur le convergent 44.

Comme on peut le voir sur les figures 2 et 3, le convergent 44 est moulé sur le module 10 de façon à être retenu par un film charnière. Ainsi, au moment de la fabrication du module 10, le convergent est orienté verticalement, de façon à permettre le démoulage facile du module 10, comme on peut le voir sur la figure 2. Ensuite, ce convergent 44 est rabattu de façon à être orienté horizontalement et exercer sa fonction de guide d'air, comme on peut le voir sur la figure 3.

Pour faciliter ce pliage du convergent, et également pour ne pas gêner la compression des jambages 16a et 16b, le convergent 44 comporte deux extrémités 46 qui ne sont pas directement solidarisées aux jambages 16a et 16b. En d'autres termes, le film charnière s'étend entre les deux jambages 16a et 16b, mais pas en-dessous des jambages où il libère les extrémités 46 par rapport aux jambages. Ainsi, en cas de chocs, les jambages 16a et 16b, ainsi que l'élément transversal inférieur 14, peuvent se déformer et reculer en direction des longerons, sans que le convergent 44, qui apporte une certaine rigidité dans la direction longitudinale X, ne constitue un obstacle à cette déformation.

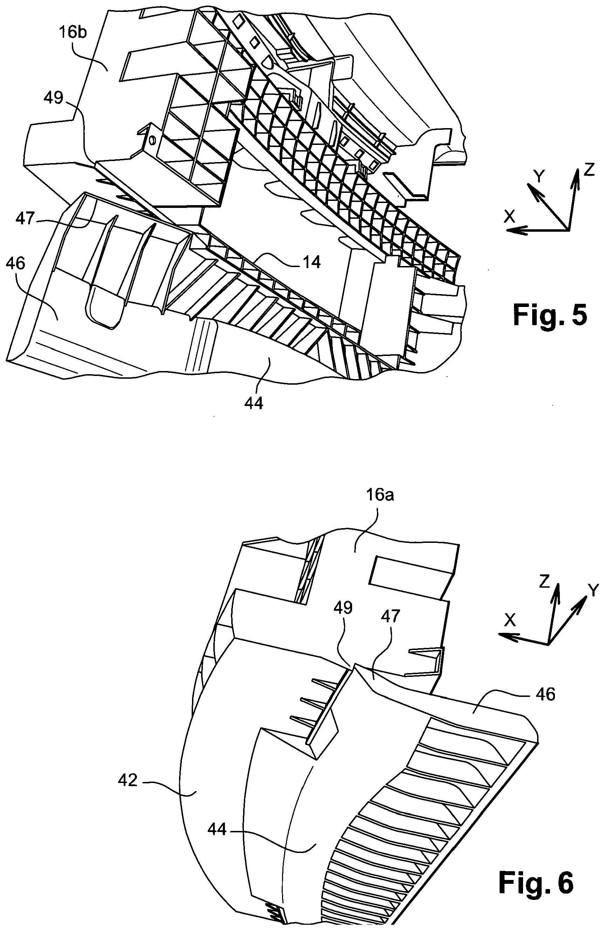

Pour permettre le maintien du convergent 44 dans sa position horizontale, et pour éviter les vibrations, on prévoit notamment, sur ses extrémités 46, des moyens 47 de calage du convergent dans la direction longitudinale X du véhicule, coopérant avec des moyens de calage complémentaires 49 ménagés sur la paroi inférieure des jambages 16a, 16b, visibles sur la figure 5. Ces moyens de calage sont capables de s'effacer en cas de choc de type assurance pour que les jambages 16a, 16b puissent se déformer et reculer dans la direction X. Ces moyens de calage se présentent sur la figure 5 sous la forme de moyens formant butée dans la direction X, mais l'on pourrait également prévoir que ces moyens forment butée dans la direction verticale Z, ce qui offrirait l'avantage de participer à l'immobilisation du convergent 44 dans sa position horizontale. De tels moyens formant une butée verticale pourraient prendre la forme d'une nervure ménagée le long et sur le bas de la butée 49, de façon à former des moyens fusibles d'immobilisation du convergent 44 dans sa position horizontale, aptes à être franchis en force en cas de choc assurance pour libérer le bas des jambages 16a, 16b et ainsi que le convergent 44 ne constitue pas un obstacle pour leur compression contre les pièces structurelles inférieures 36 ou contre des longerons inférieurs.

Le convergent 44 comporte également des nervures de rigidification en vue d'un traitement efficace du choc jambe. Il comporte en outre une traverse 48, visible sur la figure 3, rapportée ou venue de moulage, pouvant assurer une fonction de rigidité du cadre absorbeur, ou de l'avant du véhicule en général, notamment en pouvant supporter le radiateur. Cette traverse est par exemple fixée au radiateur.

On notera que le convergent 44, dans sa partie médiane située entre les deux longerons, peut également comporter des moyens d'immobilisation du convergent 44 dans sa position horizontale, aptes à maintenir le convergent 44 en position en cas de choc piéton, et à s'effacer pour permettre son recul en cas de choc assurance, afin de faciliter l'absorption par déformation du cadre absorbeur 18.

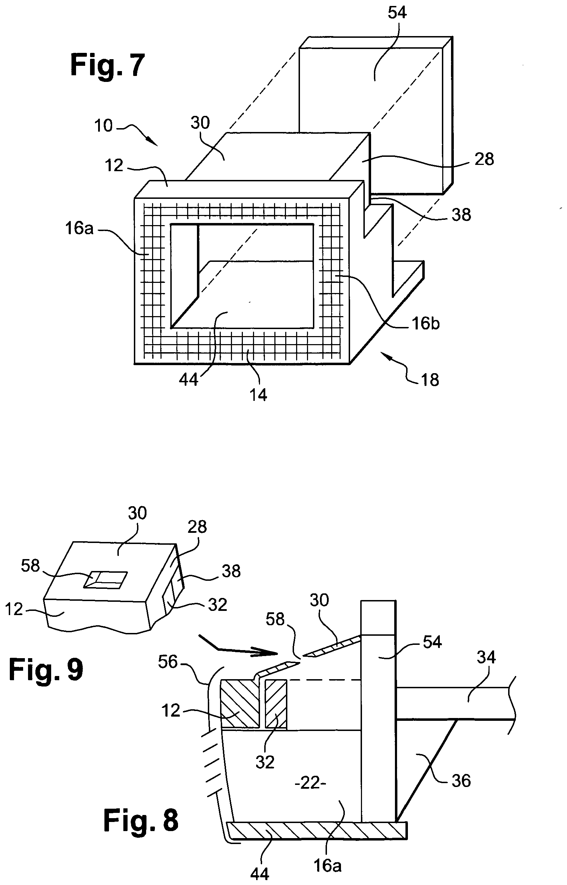

Sur le mode de réalisation des figures 7 à 9, les éléments analogues sont désignés par des références identiques.

La paroi 30 formant guide d'air s'étendant depuis l'élément transversal supérieur 12 vers le radiateur 54 n'est pas un élément d'un renfort central et a pour fonction, à l'aide dans cet exemple des parois 28 et des languettes 38, d'obturer la partie supérieure du caisson de guidage 22. Cette paroi 30 a la forme d'un voile, elle est sensiblement horizontale mais peut aussi être inclinée. De préférence, elle comporte en son centre une ouverture 58 destinée à faire passer de l'air issu d'entrées d'air supérieures de la peau de pare-chocs dans le caisson de guidage 22.

Afin d'assurer une étanchéité optimale de l'air entrant par les entrées d'air de la peau de pare-chocs, on peut prévoir un conduit allant depuis par exemple une entrée d'air supérieure de la peau jusqu'à l'ouverture 58 de la paroi 30, afin d'amener l'air vers le caisson étanche 22.

On comprendra que le caisson 22, grâce aux différents moyens de guidage 24a, 40a, 24b, 40b, 26, 28, 30, 38, est un caisson particulièrement performant pour guider de l'air entrant par des ouvertures ménagées dans la peau de pare-chocs, entrant par les ouvertures 50 ou 58 du renfort 20 ou de la paroi 30 ou bien par l'ouverture centrale du cadre 18, vers le radiateur 54 du véhicule, comme cela est schématisé sur les figures 4 et 8. En rendant les différentes jonctions entre les guides d'air étanches, on peut obtenir un caisson capable de guider l'air de façon étanche, sans fuite, ce qui procure un caisson étanche particulièrement intéressant, puisqu'il offre la possibilité, grâce à son efficacité de guidage, d'utiliser des radiateurs moins hauts, ce qui permet de réduire le porte-à-faux du véhicule. Cette étanchéité est facilitée lorsque l'ensemble de ces éléments est moulé d'un seul tenant.

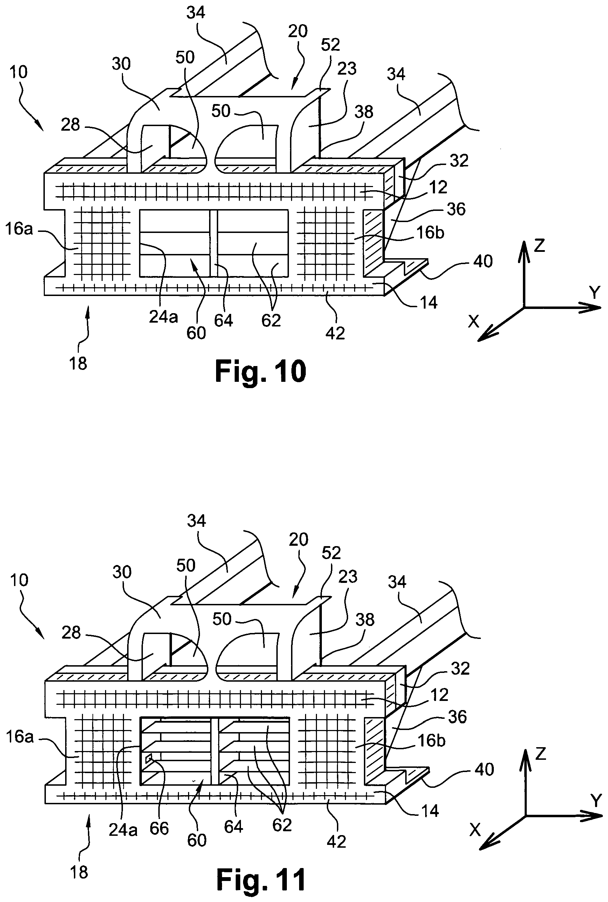

Selon un mode de réalisation représenté sur les figures 10 et 11, le module 10 comprend des moyens amovibles 60 d'obturation d'une entrée d'air ménagée dans la peau de pare-chocs, les moyens 60 étant aptes à obturer le caisson de guidage 22. Ces moyens 60 comportent des volets 62, pivotants chacun autour d'un axe longitudinal central du volet. Sur les figures 10 et 11, les axes des volets 62 sont montés chacun, d'une part sur l'une ou l'autre des parois intérieures 24a, 24b, d'autre part sur une paroi verticale 64 reliant les parties centrales avant des éléments 12 et 14.

Les volets 62 peuvent prendre une position d'obturation totale, visible sur la figure 10, dans laquelle ils définissent ensemble une surface pleine, et une ou plusieurs positions d'ouverture, dont l'une est représentée sur la figure 11. Les volets 62 sont pilotés par des moyens 66 de pilotage des volets, par exemple des moyens motorisés ou un électro-aimant. Ces moyens 66 sont déclenchés de façon automatique en fonction de la vitesse du véhicule. Dans l'exemple, les moyens 66 sont montés sur l'un ou l'autre des jambages 16a ou 16b, par exemple sur la paroi intérieure 24a ou 24b, de préférence non visibles depuis l'extérieur du véhicule.

On comprendra que des moyens similaires aux moyens 60 peuvent être utilisés sur les autres modes de réalisation du module d'absorption, et peuvent obturer tout type d'entrée d'air.

On notera que le module 10, associé à la poutre de chocs 32, peut être assemblé en dehors de la chaîne de montage du véhicule, de façon à être rapporté en un seul bloc sur le châssis, éventuellement en intégrant les moyens d'obturation 60. Il peut être fixé en divers points prévus sur les éléments 12, 14, 16a et 16b, ainsi qu'à l'aide de moyens 52 prévus sur le renfort central 20 ou sur la paroi 30, permettant de fixer le renfort ou paroi 30 sur le radiateur ou une traverse de support de ce dernier.

On notera en outre que le module 10 peut également intégrer une multiplicité de fonctions supplémentaires, telles que la fonction de support de fixations, de faisceaux, de capteurs, d'avertisseur sonore, de guidage d'air vers un autre élément que le radiateur, par exemple un radiateur d'air de suralimentation, lequel pourrait être prévu au-dessus du radiateur principal, ou encore sur les côtés, à l'extrémité extérieure de l'un des jambages 16a, 16b.

Enfin, on notera que l'invention n'est pas limitée aux modes de réalisation précédemment décrits. L'invention est définie par les revendications annexées suivantes.

En particulier, selon un mode de réalisation non décrit, le châssis arrière du véhicule ne comporte pas les pièces structurelles 36, que l'on peut appeler pendards, mais des longerons inférieurs, ou encore il comporte à la fois ces pendards 36 et des longerons inférieurs permettant de reprendre l'effort reçu par l'extrémité inférieure de ces pendards.

## Revendications

1. Ensemble d'un module (10) d'absorption de chocs pour véhicule automobile et d'une pièce structurelle inférieure (36) du véhicule automobile, le module (10) comportant :
- un élément transversal supérieur (12), destiné à être en appui contre une poutre de chocs (32) du véhicule,
- un élément transversal inférieur (14) et deux jambages (16a, 16b) reliant les éléments transversaux supérieur (12) et inférieur (14) de façon à former sensiblement un cadre (18) appelé cadre absorbeur,
**caractérisé en ce que** le module comporte une paroi (30) formant un guide d'air s'étendant depuis l'élément transversal supérieur (12) vers un radiateur (54) du véhicule, **en ce que** l'élément transversal inférieur (14) ou l'un des deux jambages (16a, 16b) est configuré pour être en appui contre la pièce structurelle inférieure (36), **en ce que** chacun des éléments transversaux supérieur (12) et inférieur (14) et chaque jambage (16a, 16b) comprend des moyens d'absorption de choc de type assurance, et **en ce que** le module (10) définit un caisson (22) de guidage de l'air vers le radiateur, délimité par la paroi intérieure (24a, 24b) de chacun des jambages (16a, 16b), la paroi supérieure (26) de l'élément transversal inférieur (14), et ladite paroi (30) formant guide d'air s'étendant depuis l'élément transversal supérieur, chacune de ces parois s'étendant jusqu'au radiateur.

2. Ensemble selon la revendication 1, dans lequel le cadre absorbeur (18) et la paroi (30) formant guide d'air sont en matière plastique, de préférence venus de moulage.

3. Ensemble selon l'une quelconque des revendications 1 à 2, dans lequel le module (10) comporte des moyens (38) de guidage de l'air destinés à être agencés entre la poutre de chocs (32) et le radiateur (54), de préférence venus de moulage avec la paroi (30) formant guide d'air s'étendant depuis l'élément transversal supérieur (12).

4. Ensemble selon l'une quelconque des revendications 1 à 3, dans lequel le caisson (22) est apte à guider l'air entrant vers le radiateur de façon étanche.

5. Ensemble selon l'une quelconque des revendications 1 à 4, dans lequel l'élément transversal inférieur (14) comprend un convergent inférieur (44), de préférence venu de moulage.

6. Ensemble selon l'une quelconque des revendications précédentes, dans lequel le module (10) comporte, au-dessus de l'élément transversal supérieur (12), un renfort central (20), de préférence un support de la peau de pare-chocs du véhicule.

7. Ensemble selon la revendication précédente, dans lequel le renfort central (20) comprend la paroi (30) formant guide d'air s'étendant depuis l'élément transversal supérieur vers le radiateur.

8. Ensemble selon l'une quelconque des revendications précédentes, dans lequel le module (10) comprend des moyens amovibles d'obturation d'une entrée d'air.

9. Ensemble selon l'une quelconque des revendications précédentes, dans lequel chaque jambage (16a, 16b) est configuré pour prendre appui contre la pièce structurelle inférieure (36) comprenant une portée d'appui, s'étendant vers le bas dans le prolongement de l'extrémité de chacun des longerons (34).

10. Ensemble selon l'une quelconque des revendications précédentes, dans lequel la pièce structurelle inférieure (36) comprend un haubanage, par exemple sous forme d'équerre.

11. Ensemble selon l'une quelconque des revendications précédentes, dans lequel la pièce structurelle inférieure (36) comprend des longerons inférieurs.

12. Ensemble d'une poutre de chocs et d'un ensemble selon l'une quelconque des revendications précédentes.

## Patentansprüche

1. Anordnung aus einem Aufpralldämpfungs-Modul (10) für Kraftfahrzeug und einem unteren Strukturteil (36) des Kraftfahrzeugs, wobei das Modul (10) Folgendes aufweist:
- ein oberes Querelement (12), das zur Abstützung gegen einen Aufprallträger (32) des Fahrzeugs bestimmt ist,
- ein unteres Querelement (14) und zwei Pfosten (16a, 16b), die das obere (12) und untere (14) Querelement so verbinden, dass sie im Wesentlichen einen Rahmen (18), als Dämpfungsrahmen bezeichnet, bilden,
**dadurch gekennzeichnet, dass** das Modul eine eine Luftführung bildende und sich von dem oberen Querelement (12) aus zu einem Kühlkörper (54) des Fahrzeugs hin erstreckende Wand (30) aufweist, dass das untere Querelement (14) oder einer der zwei Pfosten (16a, 16b) ausgestaltet ist, um gegen das untere Strukturteil (36) in Abstützung zu sein, dass jedes des oberen (12) und unteren (14) Querelements und jeder Pfosten (16a, 16b) Aufpralldämpfungsmittel des Versicherungstyps umfasst und dass das Modul (10) einen Kasten (22) zum Führen von Luft zu dem Kühlkörper hin definiert, der durch die untere Wand (24a, 24b) jedes der Pfosten (16a, 16b), die obere Wand (26) des unteren Querelements (14) und die eine Luftführung bildende und sich von dem oberen Querelement aus erstreckende Wand (30) begrenzt ist, wobei sich jede dieser Wände bis zu dem Kühlkörper erstreckt.

2. Anordnung nach Anspruch 1, wobei der Dämpfungsrahmen (18) und die eine Luftführung bildende Wand (30) aus Kunststoff sind, vorzugsweise gegossen.

3. Anordnung nach einem der Ansprüche 1 bis 2, wobei das Modul (10) Mittel (38) zum Führen von Luft aufweist, die dazu bestimmt sind, zwischen dem Aufprallträger (32) und dem Kühlkörper (54) vorgesehen zu sein, vorzugsweise mit der eine Luftführung bildenden und sich von dem oberen Querelement (12) aus erstreckenden Wand (30) gegossen.

4. Anordnung nach einem der Ansprüche 1 bis 3, wobei) der Kasten (22)in der Lage ist, die einströmende Luft auf abgedichtete Weise zu dem Kühlkörper hin zu führen.

5. Anordnung nach einem der Ansprüche 1 bis 4, wobei das untere Querelement (14) einen unteren Verjüngungsrohrstutzen (44) umfasst, vorzugsweise gegossen.

6. Anordnung nach einem der vorhergehenden Ansprüche, wobei das Modul (10), oberhalb des oberen Querelements (12), eine mittige Verstärkung (20) aufweist, vorzugsweise einen Träger der Stoßfängerhaut des Fahrzeugs.

7. Anordnung nach dem vorhergehenden Anspruch, wobei die mittige Verstärkung (20) die eine Luftführung bildende und sich von dem oberen Querelement aus erstreckende Wand (30) umfasst.

8. Anordnung nach einem der vorhergehenden Ansprüche, wobei das Modul (10) entfernbare Mittel zum Verschließen eines Lufteinlasses umfasst.

9. Anordnung nach einem der vorhergehenden Ansprüche, wobei jeder Pfosten (16a, 16b) dafür ausgebildet ist, gegen das untere Strukturteil (36) zur Abstützung zu kommen, das eine Abstützfläche umfasst, die sich nach unten in der Verlängerung des Endes jedes der Längsträger (34) erstreckt.

10. Anordnung nach einem der vorhergehenden Ansprüche, wobei das untere Strukturteil (36) eine Abspannung umfasst, zum Beispiel in Form eines Winkels.

11. Anordnung nach einem der vorhergehenden Ansprüche, wobei das Strukturteil (36) untere Längsträger umfasst.

12. Anordnung aus einem Aufprallträger und einer Anordnung nach einem der vorhergehenden Ansprüche

## Claims

1. Assembly of a shock absorption module (10) for an automobile vehicle and a lower structural part (36) of the automobile vehicle, the module (10) comprising:
- an upper transverse member (12) intended to rest against a bumper (32) of the vehicle,
- a lower transverse member (14) and two jambs (16a, 16b) connecting the upper (12) and lower (14) transverse members so as to substantially form a frame (18), called absorber frame,
**characterised in that** the module comprises a wall (30) forming an air guide extending from the upper transverse member (12) to a radiator (54) of the vehicle, **in that** the lower transverse member (14) or one of the two jambs (16a, 16b) is configured to rest against the lower structural part (36), **in that** each of the upper (12) and lower (14) transverse members and each jamb (16a, 16b) comprises absorption means of shock of insurance type, and **in that** the module (10) defines a caisson (22) for guiding air towards the radiator, delimited by the inner wall (24a, 24b) of each jamb (16a, 16b), the upper wall (26) of the lower transverse member (14) and said wall (30) forming air guide extending from the upper transverse member, each of these walls extending until the radiator.

2. Assembly according to claim 1, wherein the absorber frame (18) and the wall (30) forming air guide are made of plastic, preferably integrally moulded.

3. Assembly according to claim 1 or 2, wherein the module (10) comprises air guide means (38) intended to be arranged between the bumper (32) and the radiator (54), preferably integrally moulded with the wall (30) forming air guide extending from the upper transverse member (12).

4. Assembly according to any one of claims 1 to 3, wherein the caisson is adapted to sealably guide the incoming air towards the radiator.

5. Assembly according to any one of claims 1 to 4, wherein the lower transverse member (14) comprises a lower convergent (44), preferably integrally moulded.

6. Assembly according to any one of the preceding claims, wherein the module (10) comprises, above the upper transverse member (12), a central reinforcement (20), preferably a support for the bumper skin of the vehicle.

7. Assembly according to the preceding claim, wherein the central reinforcement (20) comprises the wall (30) forming air guide extending from the upper transverse member to the radiator.

8. Assembly according to any one of the preceding claims, wherein the module (10) comprises removable means for blocking an air inlet.

9. Assembly according to any one of the preceding claims, wherein each jamb (16a, 16b) is configured to rest against the lower structural part (36) comprising a bearing surface, extending downwards in the prolongation of the end of each of longitudinal members (34).

10. Assembly according to any one of the preceding claims, wherein the lower structural part (36) comprises a bracing, for example in the form of an angle bracket.

11. Assembly according to any one of the preceding claims, wherein the lower structural part (36) comprises lower longitudinal members.

12. Assembly of a bumper and an assembly according to any one of the preceding claims.
